# EUROPÄISCHE PATENTSCHRIFT

(11) **EP 1 264 615 B1**
(45) Veröffentlichungstag und Bekanntmachung des Hinweises auf die Patenterteilung: **24.12.2008**
(21) Anmeldenummer: 02090201.1
(22) Anmeldetag: 04.06.2002
(51) Int. Cl.: A61N 1/39

(54) **Therapiegerät**
Therapy device
Appareil de thérapie

(30) Priorität: 08.06.2001 DE 10128979
(43) Veröffentlichungstag der Anmeldung: 11.12.2002
(73) Patentinhaber: Corscience GmbH & CO.KG, 91052 Erlangen (DE)
(72) Erfinder: Bolz, Armin, Prof. Dr., 91054 Buckenhof (DE)
(74) Vertreter: Herrmann, Uwe

(56) Entgegenhaltungen:
- EP-A- 0 526 175
- EP-A- 1 099 406
- US-A- 4 273 114
- US-A- 4 424 806
- US-A- 4 928 674
- US-A- 5 490 820
- US-A- 5 626 151
- US-A- 5 683 424

## Beschreibung

Die Erfindung betrifft ein Therapiegerät für die Notfallbehandlung von Patienten, insbesondere Patienten mit akutem Herzversagen. Solche Therapiegeräte sind grundsätzlich bekannt und umfassen in der Regel eine Defibrillationseinheit und eine Beatmungseinheit, um Patienten, die an einer ventrikulären Fibrillation (VF) oder einer anderen schockbehandelbaren Tachycardie (VT) leiden, zu behandeln, indem über die Defibrillationseinheit ein Elektroschock abgegeben wird, der die Fibrillation des Herzens des Patienten unterbrechen soll und über die Beatmungseinheit die Beatmung des Patienten sichergestellt wird.

Entsprechende Verfahren zur Notfallbehandlung der Patienten ebenso wie entsprechende Geräte sind grundsätzlich bekannt, beispielsweise aus der WO 01/12122 und den US-Patenten 5,626,151 und 5,975,081.

Aus dem Europäischen Patent 0 688 201 ist außerdem ein Gerät zur Brustkorbkompression in Verbindung mit einer Liege bekannt, welches zusammen mit einem Defibrillator eingesetzt werden kann.

Die US 4,273,114 offenbart eine Wiederbelebungsvorrichtung, die automatisiert eine Herzdruckmassage mittels einer druckluftbetriebenen CPR-Druckvorrichtung durchführen kann. Ferner sind ein Defibrillator und eine Beatmungsvörrichtung beschrieben, wobei die Beatmungsvorrichtung und die Druckvorrichtung von derselben Druckluftquelle gespeist werden.

Die US 4,928,674 betrifft eine Wiederbelebungsvorrichtung, die eine am Oberkörper anzulegende Kompressionsweste für die Druckmassage, einen darin integrierten Defibrillator und eine Beatmungsvorrichtung aufweist.

Die US 5,626,151 betrifft eine Lebensrettungstrage für den Einsatz in Kriegs- bzw. Kampfgebieten, die speziell für die Versorgung von Verwundeten ausgebildet ist.

Die US 5,490,820 eine Wiederbelebungsvorrichtung, die mit einer Druckmassagevorrichtung, einer Beatmungseinheit, einem Defibrillator und einer zentralen Steuereinheit ausgestattet ist, die die vorgenannten Komponenten steuert.

Aus dem US Patent 6,125,299 ist ein tragbarer, externer Defibrillator bekannt, der außerdem Sensoren für die zur Brustkorbkompression seitens eines Helfers ausgeübte Kraft aufweist. Damit ist es möglich, dem Helfer eine Rückmeldung über die richtige oder falsche Kompressionskraft zu geben.

Alle bekannten Gräte setzen jedoch gewisse Kenntnisse eines Helfers für das Bedienen der Geräte voraus und sind nicht einfach und sicher genug in der Anwendung.

Es besteht zumindest der Wunsch nach einem Therapiegerät für die Notfallbehandlung, insbesondere von VF-Patienten, die von jedermann zu bedienen sind und an öffentlichen Orten zur Verfügung gestellt werden können. Im Falle des Notfalls kann dann auch ein nicht ausgebildeter Helfer das Therapiegerät zur Behandlung eines Patienten einsetzen, ähnlich wie jedermann einen Feuerlöscher zur Bekämpfung von Feuer einsetzen kann.

Ein solches portables Therapiegerät umfasst erfindungsgemäß eine Beatmungseinheit mit einer Beatmungsmaske für die Beatmung eines Patienten, eine Defibrillationseinheit, einen Defibrillationsdetektor und eine Steuereinheit, die mit der Beatmungseinheit, dem Defibrillationsdetektor und der Defibrillationseinheit verbunden und ausgebildet ist, die Beatmung und die Defibrillation eines Patienten nach vorgegebenen Regeln automatisch zu koordinieren. Die Steuereinheit ist bevorzugt so ausgebildet, dass sie im Einsatzfall zunächst die Beatmungseinheit zur Beatmung eines Patienten ansteuert und anschließend die Defibrillationseinheit.

Dabei ist die Steuereinheit vorzugsweise ausgebildet, nach dem Anlegen von Beatmungsmaske oder -tubus sowie Defibrillationselektroden an einen Patienten zuerst eine Beatmung auszulösen und gleichzeitig Impedanzmessung für die Steuerung der Defibrillationseinheit durchzuführen.

Ein derartiges Therapiegerät trägt erstmals der Tatsache Rechnung, dass eine Fibrillation, insbesondere eine ventrikuläre Fibrillation eine Sauerstoffunterversorgung eines Patienten zur Folge hat, die zu irreversiblen Hirnschäden führen kann. Daher ist das Therapiegerät so ausgebildet, dass ein Patient zwangsläufig zuerst beatmet wird, um den Sauerstoffgehalt des Blutes zu erhöhen. Die Erfindung beruht nämlich auf der weiteren Erkenntnis, dass auch im Falle einer ventrikulären Fibrillation noch mit einem eingeschränkten Blutfluss zu rechnen ist, der beispielweise 20% des regulären Blutflusses entspricht. Indem ein Notfallpatient zunächst beatmet wird, können Hirnschäden infolge einer ventrikulären Fibrillation wirkungsvoll eingeschränkt werden. Die bekannten Geräte weisen demgegenüber den Nachteil auf, dass eine künstliche Beatmung üblicherweise erst im Anschluss an die erste Defibrillation durchgeführt wird, wodurch kostbare Zeit bis zur Wiederherstellung der Versorgung des Organismus' mit sauerstoffreichem Blut verloren geht.

Erfindungsgemäß ist das Therapiegerät so ausgebildet, dass die Steuereinheit die Beatmung zur Fibrillationsdetektion durch den Fibrillationsdetektor unterbricht und anschließend die Defibrillationseinheit in Abhängigkeit des Ergebnisses der Fibrillationsdetektion durch den Fibrillationsdetektor ansteuert.

Dieser Gedanke, das Therapiegerät so auszubilden, dass die Beatmung zur Fibrillationsdetektion zwangsunterbrochen wird, beruht aus der Erkenntnis, dass die Beatmung die Fibrillationsdetektion stören kann. Die Zwangsunterbrechung der Beatmung kann als selbständig schutzwürdiger Gedanke auch bei solchen Therapiegeräten verwirklicht werden, bei denen die Therapie, wie aus dem Stand der Technik bekannt, mit einer Defibrillation beginnt und erst dann beatmet wird, um anschließend nach einer Fibrillationsdetektion nötigenfalls ein weiteres Mal zu defibrillieren. Die Zwangsunterbrechung der Beatmung zur Fibrillationsdetektion ermöglicht auch dem unerfahrenen Helfer die Bedienung des Therapiegerätes. Im Anschluss an die Fibrillationsdetektion wird eine Defibrillation ausgelöst.

Hierdurch wird ein wesentlicher Mangel bekannter Therapiegeräte erkannt und behoben. Die bekannten Geräte weisen Unsicherheiten bei der Detektion von Fibrillationen auf, da die ohnehin schon geringe Signalqualität der von Laien nicht fachmännisch anbringbaren EKG-Elektroden zusätzlich durch die Atmungs- bzw. künstliche Beatmungsaktivität weiter gemindert wird.

Vorzugsweise ist das Therapiegerät zur Ausgabe von Instruktionen an einen Helfer ausgebildet. Das Therapiegerät umfasst dazu vorzugsweise eine Sprachausgabeeinheit, die mit der Steuereinheit verbunden ist, um an den Helfer je nach Betriebszustand des Therapiegerätes, d.h. je nach Stadium der Therapie, adäquate Sprachinstruktionen auszugeben.

Das Therapiegerät, insbesondere die Steuereinheit, ist dabei vorzugsweise so ausgebildet, dass es einen Helfer zunächst instruiert, einem Patienten eine Beatmungsmaske anzulegen, vorzugsweise das Anlegen der Beatmungsmaske selbstständig detektiert, anschließend die Beatmung startet und den Helfer instruiert, die Defibrillationselektroden an den Patienten anzulegen. Die Defibrillationselektroden sind mit der Defibrillationseinheit verbunden. Die Defibrillationseinheit umfasst vorzugsweise einen Detektor für das Detektieren des korrekten Anliegens der Elektroden. Weiterhin ist die Steuereinheit vorzugsweise so ausgebildet, dass sie das Beatmen des Patienten entweder zeitgesteuert und/oder sobald die Defibrillationselektroden korrekt angelegt sind, unterbricht und den Fibrillationsdetektor zum Detektieren der Fibrillation ansteuert. Der Fibrillationsdetektor ist entweder direkt mit der Defibrillationseinheit oder über die Steuereinheit mit der Defibrillationseinheit verbunden, so dass nach der Fibrillationsdetektion die Defibrillationseinheit in Abhängigkeit eines Ausgangssignals des Fibrillationsdetektors angesteuert wird. Zum Unterbrechen einer Fibrillation umfasst die Detektionseinheit einen Schockgeber, der mit den Defibrillationselektroden verbunden und ausgebildet ist, über die Elektroden einen Elektroschock an den Patienten auszugeben, der geeignet ist, das Herzgewebe (Myokard) des Herzens des Patienten möglichst vollständig in einen refraktären Zustand zu versetzen, um die Fibrillation zu unterbrechen.

Weiterhin ist das Therapiegerät ausgebildet, die Beatmung des Patienten während der Abgabe des Defibrillationsschocks oder bevorzugt nach Abgabe des Defibrillationsschocks fortzusetzen und nach vorgegebener Zeit für eine erneute Fibrillationsdetektion wieder zu unterbrechen, um nötigenfalls einen weiteren Defibrillationsschock abzugeben, falls der erste nicht zu einer Unterbrechung der Fibrillation geführt haben sollte.

In einer bevorzugten Ausführungsvariante ist das Therapiegerät, insbesondere die Steuereinheit ausgebildet, Instruktionen an einen Helfer auszugeben, die Beatmungsmaske zur Beatmung des Patienten durch einen Beatmungstubus zu ersetzen, um eine wirksame Beatmung zu ermöglichen.

Das Therapiegerät ist vorzugsweise mit einer Hand tragbar ausgebildet und weist in einer besonders bevorzugten Ausführungsvariante leicht zugängliche Fächer für die Defibrillationselektroden, für einen Beatmungsschlauch, für die Beatmungsmaske und für den Beatmungstubus auf.

Die Behandlung eines Patienten mit einem derartigen Therapiegerät läuft in der Regel wie folgt ab: ein Helfer trägt das Therapiegerät zu einem Patienten und schaltet das Therapiegerät ein. Das Therapiegerät instruiert den Helfer, dem Patienten eine Beatmungsmaske anzulegen. Das Therapiegerät ermittelt das Anliegen der Beatmungsmaske entweder sensorgesteuert selbständig, oder der Helfer bestätigt das korrekte Anliegen der Beatmungsmaske. Das Therapiegerät steuert die Beatmungseinheit an, den Patienten mit leichten Sauerstoffüberdruck zu beatmen. Nach erfolgreichem Anlegen der Beatmungsmaske instruiert das Therapiegerät den Helfer, an dem Patienten die Defibrillationselektroden anzulegen. Die Defibrillationselektroden sind vorzugsweise selbstklebende Elektroden, die auf den Patienten aufgeklebt werden. Das erfolgreiche Anlegen der Defibrillationselektroden wird von dem Therapiegerät entweder sensorisch selbständig verfasst oder durch eine Eingabe des Helfers bestätigt. Nach erfolgreichem Anlegen der Defibrillationselektroden unterbricht das Therapiegerät die Beatmung und führt eine VF-Detektion durch, steuert anschließend die Defibrillationseinheit zur Abgabe eines Defibrillationsschocks an und steuert daraufhin die Beatmungseinheit zur Fortsetzung der Beatmung an. In einer bevorzugten Ausführungsvariante instruiert das Therapiegerät den Helfer, die Beatmungsmaske durch einen Beatmungstubus zu ersetzen, um den Patienten mit größeren Sauerstoffüberdruck beatmen zu können. Anschließend unterbricht das Therapiegerät die Beatmung des Patienten erneut und steuert den Fibrillationsdetektor zu einer weiteren VF-Detektion an, um den Erfolg der Schockabgabe bei der ersten Defibrillation zu überprüfen. Das Therapiegerät ist weiter so ausgebildet, dass es die Defibrillationseinheit in Abhängigkeit des Ergebnisses der Fibrillationsdetektion durch den Fibrillationsdetektor zur Abgabe eines weiteren Elektroschocks an den Patienten ansteuert. Detektiert das Therapiegerät eine erfolgreiche Defibrillation und eine selbsttätige Atmung des Patienten, gibt das Therapiegerät eine Instruktion an den Helfer aus, dass die Therapie erfolgreich abgeschlossen ist, und dass der Helfer Beatmungsmaske oder Beatmungstubus sowie die Defibrillationselektroden vom Patienten abnehmen kann.

Die Defibrillationseinheit ist vorzugsweise ausgebildet, die zwischen am Körper eines Patienten anzubringende Impedanz zu erfassen und ein entsprechendes Impedanzsignal zu bilden, wobei die Steuereinheit ausgebildet ist, das Impedanzsignal zur Steuerung der Beatmungseinheit von der Defibrillationseinheit zu übernehmen und auszuwerten.

Vorzugsweise ist außerdem die Beatmungseinheit mit einem Sensor zum Erfassen des in der Beatmungsmaske oder dem Beatmungstubus herrschenden Luftdrucks verbunden und zum Bilden eines entsprechenden Drucksignals ausgebildet. Die Steuereinheit ist dann bevorzugt ausgebildet, das Drucksignal zur Steuerung der Beatmungseinheit von der Beatmungseinheit zu übernehmen und auszuwerten.

Bei einem derartigen Therapiegerät mit einer Beatmungseinheit, die zum Abschalten der Beatmung bei Überschreitung eines vorgegebenen Granzdruckes in der Beatmungsmaske oder dem Beatmungstubus ausgebildet ist, ist die Steuereinheit vorzugsweise ausgebildet, ein Abschalten der Beatmung zu unterbinden, wenn die Auswertung des Impedanzsignals einen für eine Thoraxkompression charakteristischen Impedanzabfall anzeigt.

Ein erhebliches Problem bei der Beatmung während der Reanimation stellen die durch eine Herzdruckmassage hervorgerufenen Druckspitzen im Beatmungskanal dar. Wird der Thorax durch den Ersthelfer zusammengedrückt, wird auch die Lunge komprimiert und somit erhöht sich der Innendruck des geschlossenen Beatmungssystems. Bei an sich bekannten Beatmungsgeräten wird bei Überschreiten von 40 ― 60 mbar die Beatmung gestoppt und ein Fehlermodus eingeleitet.

Erfindungsgemäß wird die Sensorik des Defibrillators (Impedanzmessung) genutzt, um eine Herzdruckmassage zu erkennen und im Beatmungsgerät die unnötige Abschaltung zu verhindern.

Bei einer weiteren bevorzugten Ausgestaltung des Therapiegerätes kann dessen Beatmungseinheit in einem asynchronen Modus und alternativ in einem durch eine von einer natürlichen Atemtätigkeit eines Patienten gesteuerten, synchronen Demand-Modis arbeiten. Die Steuereinheit ist dabei ausgebildet, die Beatmungseinheit zwischen asynchronen Modus und Demand-Modus in Abhängigkeit von dem Impedanzsignal und/oder dem Drucksignal umzuschalten. An sich bekannte Beatmungsgeräte weisen unterschiedliche Modi auf. Dabei handelt es sich insbesondere um sogenannte Demandmodi (beatmet wird auf Anforderung, d.h. die vorhandene Atmung triggert den Beatmungsapparat) und den IPPV- Modus, bei dem asynchron ohne Berücksichtigung eventueller natürlicher Atmung künstlich beatmet wird. Der Wechsel von IPPV, der bei einer Beatmung zu Anfang sinnvoll ist, zu dem physiologischeren Demand-Modus erfolgt derzeit allgemein manuell. Eine erfindungsgemäße elektrische Kopplung von Defibrillator und Beatmungsgerät ermöglicht an dieser Stelle einen Automatismus:

Beatmet wird demnach zunächst asynchron. Über die Körperimpedanzmessung des Defibrillators (die braucht die Defibrillationseinheit ohnehin bereits für die Erkennung der Elektrodenpositionierung) oder eine zusätzliche Druckerfassung in der Beatmungsmaske wird eine natürliche Atemaktivität erkannt. Anschließend wird automatisch auf Demandbetrieb umgeschaltet. Dies hat für den Patienten erhebliche physiologische Vorteile, insbesondere ein höheres Atemminutenvolumen.

Vorzugsweise ist die Steuereinheit weiterhin ausgebildet, die Defibrillationseinheit in Abhängigkeit vom Zustand der Beatmungseinheit derart anzusteuern, dass ein Defibrillationsimpuls während einer vorgegebenen Phase im Beatmungszyklus, vorzugsweise der Expiratonsphase, ausgelöst wird um so Defibrillation und Beatmung zu synchronisieren.

Der optimale Defibrillationszeitpunkt befindet sich in der Expirationsphase, da dann die Stromdichte durch das Herz am größten ist. In diesem Fall lohnt sich also eine Information vom Beatmungsgerät an den Defibrillator mit dem Hinweis, in welchem Beatmungszustand sich der Patient befindet. Defibrillation und Beatmung sollten in dieser Form synchronisiert werden.

Die erfindungsgemäße Steuereinheit dient somit auch dem bidirektionalen Datenaustausch zwischen Beatmungseinheit und Defibrillationseinheit, um die sensorischen Eigenschaften der Defibrillationseinheit für die Beatmungseinheit (und umgekehrt) nutzen zu können.

Der Lösung der eingangs skizzierten Aufgabe dient auch eine Steuereinheit für ein Therapiegerät der vorbeschriebenen Art, die ausgebildet ist, mit einer Defibrillationseinheit oder einer Beatmungseinheit verbunden zu werden und diese so anzusteuern, dass im Einsatzfall zunächst die Beatmungseinheit zur Beatmung eines Patienten angesteuert wird, dass anschließend die Beatmung unterbrochen und eine Fibrillationsdetektion durchgeführt und dass anschließend die Defibrillationseinheit in Abhängigkeit des Ergebnisses der Defibrillationsdetektion angesteuert wird.

Die Steuereinheit ist vorzugsweise weiterhin so ausgebildet, dass sie Instruktionen in Abhängigkeit vom jeweils aktuellen Betriebszustand auslöst.

Weiterhin ist die Steuereinheit vorzugsweise mit Sensoren für das korrekte Anliegen von Defibrillationselektroden verbindbar und ausgebildet, das Auslösen einer Defibrillation zu unterbinden, solange die Steuereinheit nicht ein korrektes Anliegen der Defibrillationselektroden detektiert.

Die Beatmungseinheit ist vorzugsweise als an sich bekanntes cpap-Gerät (continuous positive airway pressure Gerät) ausgebildet. Ein derartiges Atmungsunterstützungsgerät arbeitet zur Beatmung des Patienten mit Umgebungsluft, die dem Patienten mittels einer Pumpe mit geringem Überdruck zugeführt wird. Durch die Verwendung von Umgebungsluft anstelle von sonst auch verwendetem Sauerstoff wird die Gefahr von Verbrennungen erheblich gemindert.

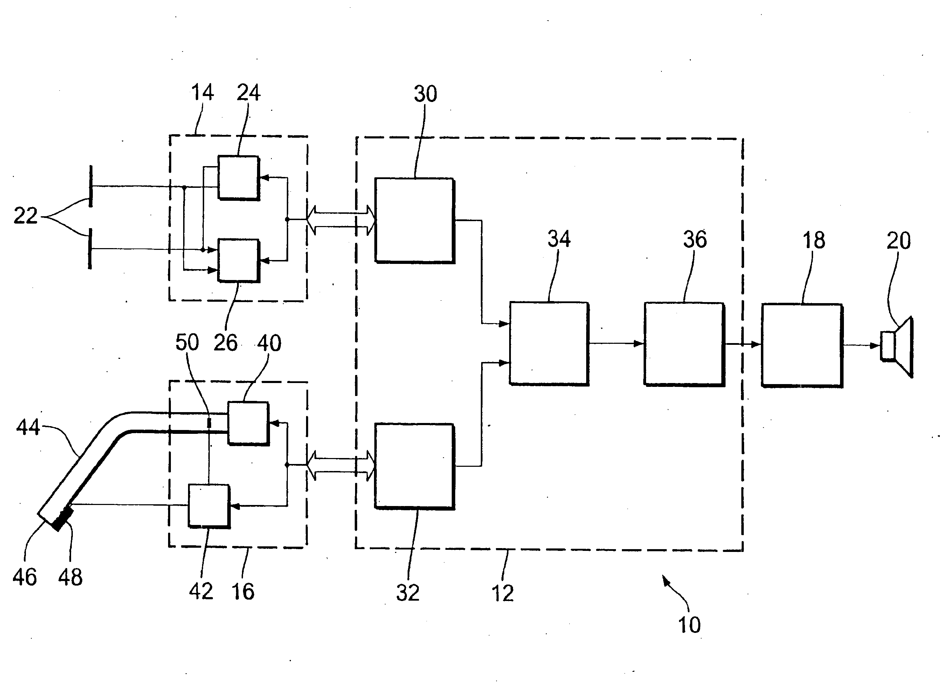

Die Erfindung soll nun anhand eines Ausführungsbeispieles mit Hilfe der Figur näher erläutert werden. Die Figur zeigt ein Therapiegerät 10 in schematischer Darstellung. Das Therapiegerät 10 umfasst als wesentliche Bestandteile eine Steuereinheit 12, die über jeweils einen Datenbus mit einer Defibrillationseinheit 14 und einer Beatmungseinheit 16 verbunden ist. Außerdem ist die Steuereinheit 12 mit einer Sprachausgabeeinheit 18 verbunden, welche einen Lautsprecher 20 umfasst.

Die Defibrillationseinheit 14 ist mit zwei Defibrillationselektroden 22 verbunden und umfasst eine Detektionseinheit 24 sowie einen Schockgeber 26. Der Schockgeber 26 ist mit den beiden Defibrillationselektroden 22 verbunden und ausgebildet, über diese Elektroden Elektroschocks an einen Patienten abzugeben. Die Detektionseinheit 24 ist ebenfalls mit den beiden Defibrillationselektroden 22 verbunden und ausgebildet, über die Defibrillationselektroden 22 elektrische Signale aufzunehmen, insbesondere ein Elektrokardiogramm, und anhand dieser Signale eine Fibrillationsdetektion durchzuführen, insbesondere eine Detektion einer ventrikulären Fibrillation (VF). Sowohl die Detektionseinheit 24 als auch der Schockgeber 26 sind mit einer Defibrillationsansteuereinheit 30 der Steuereinheit 12 verbunden. Detektionseinheit 24 und Schockgeber 26 wirken vorzugsweise derart zusammen, dass ein Elektroschock zu einem physiologisch günstigen Zeitpunkt abgegeben wird.

Die Beatmungseinheit 16 enthält eine Beatmungspumpe 40 sowie einen Beatmungssensor 42. Die Beatmungspumpe 40 ist mit einem Beatmungsschlauch 44 verbunden, an dessen freies Ende 46 eine Beatmungsmaske oder ein Beatmungstubus angeschlossen werden kann. In der Nähe des freien Endes 46 des Schlauches 44 ist ein Berührungssensor 48 vorgesehen, der auf das Anschließen eines Beatmungstubusses oder einer Beatmungsmaske anspricht. Der Berührungssensor 46 ist mit dem Beatmungssensor 42 verbunden. Weiterhin ist der Beatmungssensor 42 mit einem Luftstromsensor 50 verbunden, der im Beatmungsschlauch 44 angeordnet ist. Der Beatmungssensor 42 dient der Detektion einer natürlichen Atmungstätigkeit des Patienten über den Luftstromsensor 50 und der Kontrolle der ordnungsgemäßen Funktion der Beatmungspumpe 40 und des ordnungsgemäßen Anschlusses einer Beatmungsmaske oder eines Beatmungstubusses. Steuersignale von dem Beatmungssensor 42 und Ansteuerungssignale für die Beatmungspumpe 40 werden über einen Bus von und zu einer Beatmungsansteuereinheit 32 der Steuereinheit 12 geleitet.

Die Steuereinheit 12 umfasst neben der bereits erwähnten Defibrillationsansteuereinheit 30 und der Beatmungsansteuereinheit 32 eine Behandlungsablaufsteuerung 34, die sowohl mit der Defibrillationsansteuereinheit 30 als auch der Beatmungsansteuereinheit 32 als auch mit einer Instruktionseinheit 36 verbunden ist und ausgebildet ist, das Therapiegerät 10, und insbesondere die Defibrillationseinheit 14 sowie die Beatmungseinheit 16 über die Defibrillationsansteuereinheit 30 bzw. die Beatmungsansteuereinheit 32 so anzusteuern, dass sich der vorbeschriebene Behandlungsablauf ergibt. Je nach Betriebszustand des Therapiegerätes 10 entsprechend einem Stadium des Behandlungsablaufes steuert die Behandlungsablaufssteuerung 34 die Instruktionseinheit 36 zur Abgabe vorgegebener Instruktionen an. Diese Instruktionen werden über die Sprachausgabeeinheit 18, die mit der Instruktionseinheit 36 verbunden ist, und den Lautsprecher 20 ausgegeben.

Die Behandlungsablaufsteuerung 34 kann als Mikroprozessorsteuerung ausgebildet sein, die einen Programmspeicher für ein Programm zur Ablaufsteuerung umfasst. Alternativ kann die Ablaufsteuerung auch aus Hardwarekomponenten in einer für den Fachmann an sich bekannten Weise so aufgebaut sein, dass sich der eingangs beschriebene Steuerungsablauf ergibt. Zur bereits beschriebenen Auswertung eines von der Defibrillationseinheit stammenden Impedanzsignals zur Steuerung der Beatmungseinheit kann die Ablaufsteuerung eine Impedanzauswertungseinheit umfassen, die entweder in Form von Hardware oder als Software-Modul realisiert ist. Entsprechendes gilt für die Auswertung des von der Beatmungseinheit stammenden Drucksignals.

Das Therapiegerät 10 ist in einem portablen Gehäuse mit autarker Energieversorgung untergebracht. Das in der Figur nicht dargestellte Gehäuse umfasst Fächer für die Defibrillationselektroden 22 und Anschlusskabel von den Defibrillationselektroden 22 zu der Defibrillationseinheit 14 sowie für den Beatmungsschlauch 44 und mindestens eine Beatmungsmaske sowie mindestens einen Beatmungstubus. In der Figur nicht dargestellt ist ein Drucksensor für die Bestimmung des Luftdrucks im Inneren des Beatmungsschlauches 44, der die eingangsbeschrieben bevorzugten Betriebsmodi ermöglicht. Ein solcher Drucksensor ist ebenfalls elektrisch mit der Beatmungseinheit 16 verbunden.

## Patentansprüche

1. Portables Therapiegerät mit einer Beatmungseinheit mit Beatmungsmaske oder -tubus zur Beatmung eines Patienten, sowie mit einer Defibrillationseinheit, einem Fibrillationsdetektor und einer Steuereinheit, die mit der Beatmungseinheit und der Defibrillationseinheit verbunden und ausgebildet ist, die Ansteuerung von Beatmungseinheit und Defibrillationseinheit automatisch zu koordinieren,
**dadurch gekennzeichnet,**
**dass** die Steuereinheit ausgebildet ist, die Beatmung zur VF-Detektion durch den Fibrillationsdetektor zu unterbrechen und in Abhängigkeit der VF-Detektion die Defibrillationseinheit anzusteuern.

2. Therapiegerät nach Anspruch 1, **dadurch gekennzeichnet, dass** die Steuereinheit ausgebildet ist, zuerst die Beatmungseinheit zur Beatmung eines Patienten anzusteuern.

3. Therapiegerät nach einem der Ansprüche 1 oder 2, **dadurch gekennzeichnet, dass** das Therapiegerät zur Ausgabe von Instruktionen an einen Helfer ausgebildet ist.

4. Therapiegerät nach Anspruch 3, **dadurch gekennzeichnet, dass** das Therapiegerät eine Sprachausgabeeinheit zur Ausgabe von Instruktionen umfasst, die mit der Steuereinheit verbunden ist.

5. Therapiegerät nach einem der Ansprüche 1 oder 2, **dadurch gekennzeichnet, dass** das Therapiegerät Sensoren umfasst oder mit Sensoren verbindbar ist, die ausgebildet sind, das Anliegen einer Beatmungsmaske, das Einführen eines Beatmungstubus und/oder das Anliegen von Defibrillationselektroden zu überprüfen, wobei die Sensoren mit der Steuereinheit verbindbar sind und die Steuereinheit zur Ausgabe eines Signals ausgebildet ist, falls die Beatmungsmaske nicht korrekt angelegt, der Beatmungstubus nicht korrekt eingeführt und/oder die Defibrillationselektroden nicht korrekt angelegt sind.

6. Therapiegerät nach einem der vorhergehenden Ansprüche, bei dem die Defibrillationseinheit ausgebildet ist, die zwischen am Körper eines Patienten anzubringende Impedanz zu erfassen und ein entsprechendes Impedanzsignal zu bilden, **dadurch gekennzeichnet, dass** die Steuereinheit ausgebildet ist, das Impedanzsignal zur Steuerung der Beatmungseinheit von der Defibrillationseinheit zu übernehmen und auszuwerten.

7. Therapiegerät nach einem der vorhergehenden Ansprüche, bei dem die Beatmungseinheit mit einen Sensor zum Erfassen des in der Beatmungsmaske oder dem Beatmungstubus herrschenden Luftdrucks verbunden und zum Bilden eines entsprechenden Drucksignals ausgebildet ist, **dadurch gekennzeichnet, dass** die Steuereinheit ausgebildet ist, das Drucksignal zur Steuerung der Beatmungseinheit von der Beatmungseinheit zu übernehmen und auszuwerten.

8. Therapiegerät nach Anspruch 6 und 7, mit einer Beatmungseinheit, die zum Abschalten der Beatmung bei Überschreitung eines vorgegebenen Granzdruckes in der Beatmungsmaske oder dem Beatmungstubus ausgebildet ist, **dadurch gekennzeichnet, dass** die Steuereinheit ausgebildet ist, ein Abschalten der Beatmung zu unterbinden, wenn die Auswertung des Impedanzsignals einen für eine Thoraxkompression charakteristischen Impedanzabfall anzeigt.

9. Therapiegerät nach einem der Ansprüche 6 bis 8, deren Beatmungseinheit in einem asynchronen Modus und alternativ in einem durch eine von einer natürlichen Atemtätigkeit eines Patienten gesteuerten, synchronen Demand-Modis arbeiten kann, **dadurch gekennzeichnet, dass** die Steuereinheit ausgebildet ist, die Beatmungseinheit zwischen asynchronen Modus und Demand-Modus in Abhängigkeit von dem Impedanzsignal und/oder dem Drucksignal umzuschalten.

10. Therapiegerät nach einem der vorhergehenden Ansprüche, **dadurch gekennzeichnet, dass** die Steuereinheit ausgebildet ist, die Defibrillationseinheit in Abhängigkeit vom Zustand der Beatmungseinheit derart anzusteuern, dass ein Defibrillationsimpuls während einer vorgegebenen Phase im Beatmungszyklus, vorzugsweise der Expirationsphase, ausgelöst wird um so Defibrillation und Beatmung zu synchronisieren.

11. Therapiegerät nach einem der vorhergehenden Ansprüche, **dadurch gekennzeichnet, dass** die Steuereinheit ausgebildet ist, nach dem Anlegen von Beatmungsmaske oder -tubus sowie Defibrillationselektroden an einen Patienten zuerst eine Beatmung auszulösen und gleichzeitig Impedanzmessung für die Steuerung der Defibrillationseinheit durchzuführen.

12. Therapiegerät nach einem der vorhergehenden Ansprüche, **dadurch gekennzeichnet, dass** die Beatmungseinheit als cpap-Gerät (continuous positive airway pressure Gerät) ausgebildet ist.

13. Steuereinheit für ein Therapiegerät nach einem der vorhergehenden Ansprüche, **dadurch gekennzeichnet, dass** die Steuereinheit ausgebildet ist, mit einer Defibrillationseinheit und einer Beatmungseinheit verbunden zu werden und diese so anzusteuern, dass im Einsatzfall zunächst die Beatmungseinheit zur Beatmung eines Patienten angesteuert wird, dass anschliessend die Beatmung unterbrochen wird und eine Fibrillationsdetektion durchgeführt wird und dass anschliessend in Abhängigkeit des Ergebnisses der Defibrillationsdetektion die Defibrillationseinheit angesteuert wird.

14. Steuereinheit nach Anspruch 13, **dadurch gekennzeichnet, dass** die Steuereinheit zum Auslösen der Abgabe von Instruktionen in Abhängigkeit vom jeweils aktuellen Betriebszustand ausgebildet ist.

15. Steuereinheit nach Anspruch 13 oder 14, **dadurch gekennzeichnet, dass** die Steuereinheit mit Sensoren für das korrekte Anliegen von Defibrillationselektroden verbindbar und ausgebildet ist, das Auslösen einer Defibrillation zu unterbinden, solange die Steuereinheit nicht ein korrektes Anliegen der Defibrillationselektroden detektiert.

## Claims

1. Portable therapy device, with a breathing unit with breathing mask or tube for ventilating a patient, and with a defibrillation unit, a fibrillation detector, and a control unit that is connected to the breathing unit and to the defibrillation unit and is designed to automatically coordinate the control of breathing unit and defibrillation unit, **characterized in that** the control unit is designed to interrupt the ventilation for VF detection by the fibrillation detector and to control the defibrillation unit as a function of the VF detection.

2. Therapy device according to Claim 1, **characterized in that** the control unit is designed to first control the breathing unit for ventilation of a patient.

3. Therapy device according to one of Claims 1 and 2, **characterized in that** the therapy device is designed to output instructions to a helper.

4. Therapy device according to Claim 3, **characterized in that** the therapy device comprises a speech output unit, which outputs instructions and is connected to the control unit.

5. Therapy device according to one of Claims 1 and 2, **characterized in that** the therapy device comprises sensors or can be connected to sensors that are designed to check the fit of a breathing mask, the insertion of a breathing tube and/or the fit of defibrillation electrodes, which sensors can be connected to the control unit, and the control unit is designed to output a signal if the breathing mask is not correctly fitted, the breathing tube is not correctly inserted and/or the defibrillation electrodes are not correctly fitted.

6. Therapy device according to one of the preceding claims, in which the defibrillation unit is designed to detect the impedance to be applied to the body of a patient and to form a corresponding impedance signal, **characterized in that** the control unit is designed to take the impedance signal from the defibrillation unit and evaluate it in order to control the breathing unit.

7. Therapy device according to one of the preceding claims, in which the breathing unit is connected to a sensor for detecting the air pressure prevailing in the breathing mask or breathing tube and is designed to form a corresponding pressure signal, **characterized in that** the control unit is designed to take the pressure signal from the breathing unit and evaluate it in order to control the breathing unit.

8. Therapy device according to Claims 6 and 7, with a breathing unit that is designed to switch off the ventilation when a predetermined limit pressure is exceeded in the breathing mask or breathing tube, **characterized in that** the control unit is designed to suppress the switching-off of the ventilation if the evaluation of the impedance signal indicates an impedance drop that is characteristic of thorax compression.

9. Therapy device according to one of Claims 6 to 8, in which the breathing unit can operate in an asynchronous mode and, alternatively, in a synchronous demand mode controlled by a natural breathing activity of a patient, **characterized in that** the control unit is designed to switch the breathing unit between asynchronous mode and demand mode as a function of the impedance signal and/or the pressure signal.

10. Therapy device according to one of the preceding claims, **characterized in that** the control unit is designed to control the defibrillation unit as a function of the status of the breathing unit in such a way that a defibrillation impulse is triggered during a predetermined phase in the breathing cycle, preferably the expiration phase, in order thereby to synchronize defibrillation and ventilation.

11. Therapy device according to one of the preceding claims, **characterized in that**, after a breathing mask or tube and defibrillation electrodes have been fitted to a patient, the control unit is designed to first trigger a ventilation and at the same time to perform impedance measurement for control of the defibrillation unit.

12. Therapy device according to one of the preceding claims, **characterized in that** the breathing unit is designed as a cpap device (continuous positive airway pressure device).

13. Control unit for a therapy device according to one of the preceding claims, **characterized in that** the control unit is designed to be connected to a defibrillation unit and to a breathing unit and to control these such that, during use, the breathing unit is first controlled in order to ventilate a patient, that the ventilation is then interrupted and a fibrillation detection performed, and that the defibrillation unit is then controlled as a function of the result of the defibrillation detection.

14. Control unit according to Claim 13, **characterized in that** the control unit is designed to trigger the output of instructions as a function of the actual operating state.

15. Control unit according to Claim 13 or 14, **characterized in that** the control unit can be connected to sensors for correct fitting of defibrillation electrodes and is designed to suppress the triggering of defibrillation if the control unit does not detect a correct fit of the defibrillation electrodes.

## Revendications

1. Appareil de thérapie portable avec une unité respiratoire avec masque ou tube respiratoire pour la respiration d'un patient, et avec une unité de défibrillation, un détecteur de fibrillation et une unité de commande qui est reliée à l'unité respiratoire et à l'unité de défibrillation et est réalisée pour coordonner automatiquement la commande de l'unité respiratoire et de l'unité de défibrillation,
**caractérisé**
**en ce que** l'unité de commande est réalisée pour interrompre la respiration pour la détection VF par le détecteur de fibrillation et pour commander l'unité de défibrillation en fonction de la détection VF.

2. Appareil de thérapie selon la revendication 1, **caractérisé en ce que** l'unité de commande est réalisée pour commander d'abord l'unité respiratoire pour la respiration d'un patient.

3. Appareil de thérapie selon l'une des revendications 1 ou 2, **caractérisé en ce que** l'appareil de thérapie est réalisé pour émettre des instructions à une aide.

4. Appareil de thérapie selon la revendication 3, **caractérisé en ce que** l'appareil de thérapie comprend une unité d'émission vocale pour l'émission d'instructions, qui est reliée à l'unité de commande.

5. Appareil de thérapie selon l'une des revendications 1 ou 2, **caractérisé en ce que** l'appareil de thérapie comprend des capteurs ou peut être relié à des capteurs qui sont réalisés pour vérifier l'application d'un masque respiratoire, l'introduction d'un tube respiratoire et/ou l'application d'électrodes de défibrillation, où les capteurs peuvent être reliés à l'unité de commande, et l'unité de commande est réalisée pour l'émission d'un signal dans le cas où le masque respiratoire n'est pas appliqué correctement, le tube respiratoire n'est pas inséré correctement et/ou les électrodes de défibrillation ne sont pas appliquées correctement.

6. Appareil de thérapie selon l'une des revendications précédentes, dans lequel l'unité de défibrillation est réalisée pour détecter l'impédance à appliquer au corps d'un patient et pour former un signal d'impédance correspondant, **caractérisé en ce que** l'unité de commande est réalisée pour reprendre et évaluer de l'unité de défibrillation le signal d'impédance pour la commande de l'unité respiratoire.

7. Appareil de thérapie selon l'une des revendications précédentes, dans lequel l'unité respiratoire est reliée à un capteur pour la détection de la pression d'air régnant dans le masque respiratoire ou le tube respiratoire et est réalisée pour former un signal de pression correspondant, **caractérisé en ce que** l'unité de commande est réalisée pour reprendre le signal de pression pour la commande de l'unité respiratoire de l'unité respiratoire et pour l'évaluer.

8. Appareil de thérapie selon la revendication 6 et 7, avec une unité respiratoire qui est réalisée pour la mise hors service de la respiration lors d'un dépassement d'une pression limite prédéfinie dans le masque respiratoire ou le tube respiratoire, **caractérisé en ce que** l'unité de commande est réalisée pour empêcher une mise hors service de la respiration lorsque l'évaluation du signal d'impédance indique une chute d'impédance caractéristique d'une compression du thorax.

9. Appareil de thérapie selon l'une des revendications 6 à 8, dont l'unité respiratoire peut fonctionner selon un mode asynchrone et alternativement selon un mode de demande synchrone, commandé par une activité respiratoire naturelle d'un patient, **caractérisé en ce que** l'unité de commande est réalisée pour commuter l'unité respiratoire entre un mode asynchrone et un mode de demande en fonction du signal d'impédance et/ou du signal de pression.

10. Appareil de thérapie selon l'une des revendications précédentes, **caractérisé en ce que** l'unité de commande est réalisée pour commander l'unité de défibrillation en fonction de l'état de l'unité respiratoire de telle sorte qu'une impulsion de défibrillation est déclenchée pendant une phase prédéfinie dans le cycle respiratoire, de préférence la phase d'expiration, pour synchroniser ainsi la défibrillation et la respiration.

11. Appareil de thérapie selon l'une des revendications précédentes, **caractérisé en ce que** l'unité de commande est réalisée pour déclencher, après l'application du masque ou tube respiratoire et d'électrodes de défibrillation à un patient, d'abord une respiration et d'exécuter en même temps une mesure d'impédance pour la commande de l'unité de défibrillation.

12. Appareil de thérapie selon l'une des revendications précédentes, **caractérisé en ce que** l'unité respiratoire est réalisée comme appareil cpap (ventilation spontanée avec pression expiratoire positive).

13. Unité de commande pour un appareil de thérapie selon l'une des revendications précédentes, **caractérisée en ce que** l'unité de commande est réalisée pour être reliée à une unité de défibrillation et une unité respiratoire et pour commander celles-ci de façon qu'en cas d'utilisation, tout d'abord l'unité respiratoire pour la respiration d'un patient soit commandée, qu'ensuite la respiration soit interrompue et une détection de fibrillation soit exécutée et qu'ensuite, en fonction du résultat de la détection de défibrillation, l'unité de défibrillation soit commandée.

14. Unité de commande selon la revendication 13, **caractérisée en ce que** l'unité de commande est réalisée pour le déclenchement de l'émission d'instructions en fonction de l'état de fonctionnement respectivement actuel.

15. Unité de commande selon la revendication 13 ou 14, **caractérisée en ce que** l'unité de commande peut être reliée à des capteurs pour l'application correcte d'électrodes de défibrillation et est réalisée pour empêcher le déclenchement d'une défibrillation aussi longtemps que l'unité de commande ne détecte pas une application correcte des électrodes de défibrillation.
